(19) Europäisches Patentamt / European Patent Office / Office européen des brevets

(11) **EP 3 808 855 A1**

(12) **EUROPEAN PATENT APPLICATION**
published in accordance with Art. 153(4) EPC

(43) Date of publication:
**21.04.2021 Bulletin 2021/16**

(21) Application number: **19821933.9**

(22) Date of filing: **17.06.2019**

(51) Int Cl.:
*C12Q 1/02* (2006.01)    *C12N 1/00* (2006.01)

(86) International application number:
**PCT/JP2019/023962**

(87) International publication number:
**WO 2019/244853 (26.12.2019 Gazette 2019/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.06.2018 JP 2018115655**

(71) Applicants:
• **Shimadzu Corporation**
  **Kyoto-shi**
  **Kyoto 604-8511 (JP)**
• **Tokyo Electron Limited**
  **Tokyo**
  **107-6325 (JP)**

(72) Inventors:
• **SUZUKI, Takashi**
  **Kyoto-shi, Kyoto 604-8511 (JP)**
• **TOYODA, Kenichi**
  **Kyoto-shi, Kyoto 604-8511 (JP)**
• **TAKAHASHI, Masatoshi**
  **Kyoto-shi, Kyoto 604-8511 (JP)**
• **HARA, Keisuke**
  **Tokyo 107-6325 (JP)**
• **NAGATA, Tomohisa**
  **Sapporo-shi, Hokkaido 060-0051 (JP)**
• **OSHIMA, Yasuhiro**
  **Nirasaki-shi, Yamanashi 407-0192 (JP)**

(74) Representative: **Müller-Boré & Partner**
**Patentanwälte PartG mbB**
**Friedenheimer Brücke 21**
**80639 München (DE)**

(54) **EVALUATION METHOD FOR GENOMIC ABNORMALITIES IN CELLS**

(57)    Provided is a non-invasive evaluation method for genomic abnormality in cells, the method being simple and quick and not requiring expertise for the evaluation. Disclosed is a method for evaluating if there are genomic abnormalities in cells being tested, the cells being tested being pluripotent stem cells cultivated in a culture medium or cells resulting from the induced differentiation of pluripotent stem cells, the method including a step of measuring the amount of an indicator present in the culture supernatant of the test cells, and a step of evaluating, on the basis of the amount of the indicator present, if there are genomic abnormalities in the test cells, wherein the indicator is at least one selected from the group consisting of deoxycytidine, kynurenine, putrescine, alanine, cysteine, cystathionine, and threonic acid, and whether or not there are genomic abnormalities in the test cells is evaluated on the basis of the amount of the at least one indicator present.

FIG. 7

**Description**

[Technical Field]

The present invention relates to an evaluation method for genomic abnormalities in cells, and more specifically, to a simple, rapid and non-invasive evaluation method for genomic abnormalities in cells without requiring expertise in the evaluation.

[Background Art]

Pluripotent stem cells such as iPS cells and ES cells can differentiate into any cell types constituting the body, and are attracting a great deal of attention as a cell supply source for regenerative medicine. In order to use these cells for regenerative medicine, it is necessary to culture a large amount of iPS cells and ES cells. On the other hand, it is known that repeated division of these cells increases the risk of genomic abnormalities [chromosome numerical abnormalities, chromosome morphological abnormalities (abnormalities in which a part of a chromosome moves to another chromosome, and abnormalities in which the orientation of a part of a chromosome is reversed)] (Non-Patent Literature 1 and 2). Since cells with genomic abnormalities are more likely to cause canceration, it is necessary to check whether these genomic abnormalities occur before cell transplantation.

As a method of analyzing chromosomes, a method in which, after a certain number of cells are cultured, division is stopped with a colcemid solution, the cells are treated with a hypotonic solution, and then fixed with methanol/acetic acid, and then a specimen is prepared on a slide glass, a Giemsa staining or G band/Q band treatment is performed, and observation under a microscope is then performed is general. Refer to Non-Patent Literature 3 (the Japanese Association for Chromosome and Gene Analysis Vol. 32 No. 1 2014, pp 60-89).

In JP2010-508815 (WO2008/066655), a method in which components in a culture medium when oocytes are cultured for fertilization and implantation are analyzed, and chromosome abnormalities are diagnosed is disclosed. In this literature, target cells are oocytes, and there is no disclosure for pluripotent stem cells.

[Citation List]

[Patent Literature]

[Patent Literature 1]
JP2010-508815
[Patent Literature 2]
WO2008/066655

[Non-Patent Literature]

[Non-Patent Literature 1]
Louise C. Laurent, et. al., "Dynamic Changes in the Copy Number of Pluripotency and Cell Proliferation Genes in Human ES and iPS Cells during Reprogramming and Time in Culture," Cell Stem Cell 8, 106-118, January 7, 2011
[Non-Patent Literature 2]
"Screening ethnically diverse human embryonic stem cells identifies a chromosome 20 minimal amplicon conferring growth advantage." Nature Biotechnology volume 29, 1132-1144 (2011)
[Non-Patent Literature 3]
The Official Journal of the Japanese Association for Chromosome and Gene Analysis, "Guidelines for Quality Assurance of Chromosomal and Genetic Testing Second Edition," the Japanese Association for Chromosome and Gene Analysis Vol. 32 No. 1 2014, pp 60-89

[Summary of Invention]

[Technical Problem]

The chromosome analysis method disclosed in Non-Patent Literature 3 requires expertise for evaluation because procedures including a pretreatment operation are complicated and also visual observation under a microscope is necessary. Therefore, analysis and evaluation by outsourcing are general. In this case, several weeks to several months are required to obtain analysis results.

In addition, in all of the above analysis methods, it is necessary to perform invasive treatment on cells. Therefore, after chromosomes are analyzed, it is not possible to use the cells used for the evaluation for another purpose, for example, as a cell source for regenerative medicine.

An objective of the present invention is to provide a simple, rapid and non-invasive evaluation method for genomic abnormalities in cells without requiring expertise in the evaluation.

[Solution to Problem]

The inventors conducted extensive studies, and as a result, completed the present invention by finding that it is possible to evaluate genomic abnormalities in cells based on the abundance of a specific indicator in a culture supernatant.

The present invention includes the following inventions.

(1) An evaluation method for genomic abnormalities, wherein is evaluated whether test cells have genomic abnormalities using pluripotent stem cells cultured in a culture medium or cells induced to differentiate from pluripotent stem cells as the test cells, the method including: a step of measuring an abundance of an indicator in a culture supernatant of test cells; and a step of evaluating whether the test cells have genomic abnormalities based on the abundance of the indicator, wherein the indicator is at least one selected from the group consisting of deoxycytidine, kynurenine, putrescine, alanine, cysteine, cystathionine, and threonic acid, and wherein it is evaluated whether the test cells have genomic abnormalities based on the abundance of the at least one indicator.

(2) The evaluation method for genomic abnormalities according to (1), further including a step of measuring an abundance of the indicator in a culture supernatant of control cells known to have normal chromosomes, wherein, for the at least one indicator, it is evaluated whether the test cells have genomic abnormalities by comparing the abundance of the indicator in the culture supernatant of the test cells with the abundance of the indicator in the culture supernatant of the control cells.

(3) The evaluation method for genomic abnormalities according to (2), wherein, for the at least one indicator, based on determination of whether the ratio of the abundance of the indicator in the culture supernatant of the test cells to the abundance of the indicator in the culture supernatant of the control cells is at a predetermined threshold value or more, or at the predetermined threshold value or less, it is evaluated whether the test cells have genomic abnormalities.

(4) The evaluation method for genomic abnormalities according to (3), wherein, when the indicator is deoxycytidine, kynurenine, putrescine, cystathionine, or threonic acid, it is evaluated whether the test cells have genomic abnormalities based on determination of whether the ratio is at the predetermined threshold value or less.

(5) The evaluation method for genomic abnormalities according to claim 3, wherein, when the indicator is alanine or cysteine, it is evaluated whether the test cells have genomic abnormalities based on determination of whether the ratio is at the predetermined threshold value or more.

(6) The evaluation method for genomic abnormalities according to any one of (1) to (4), wherein among the indicators, at least one selected from the group consisting of putrescine, deoxycytidine, and kynurenine is selected as the indicator.

(7) The evaluation method for genomic abnormalities according to any one of (1) to (6), wherein among the indicators, two or more indicators are selected.

(8) The evaluation method for genomic abnormalities according to any one of (1) to (7), wherein it is evaluated whether there are abnormalities on the 18th chromosome.

(9) The evaluation method for genomic abnormalities according to any one of (1) to (8), wherein the abundance of the indicator in the culture supernatant is evaluated by liquid chromatography-mass spectrometry (LC-MS).

A cell culture method including a process of selecting the test cells based on the results of evaluating genomic abnormalities by the method according to any one of (1) to (9).

In the evaluation method for genomic abnormalities of the present invention, pluripotent stem cells cultured in a culture medium or cells induced to differentiate from pluripotent stem cells are used as the test cells. However, without being limited to pluripotent stem cells, the method can also be applied to general cells cultured in a culture medium. The pluripotent stem cells include embryonic stem cells (ES cells), induced pluripotent stem cells (iPS cells), and the like.

In the present invention, the abundance of the indicator in the culture supernatant may be measured using any method. Representative methods include, for example, a gas chromatographic analysis method (GC), a liquid chromatographic analysis method (LC), mass spectrometry (MS), liquid chromatography-mass spectrometry (LC-MS), and gas chromatography-mass spectrometry (GC-MS).

[Advantageous Effects of Invention]

According to the present invention, since it is only necessary to collect the culture supernatant for analyzing chromosomes of cells, it is possible to obtain analysis results in a short time without requiring complicated pretreatment as in the related art. In addition, since it is not necessary to perform invasive treatment on cells as in the related art, it is possible to use test cells as a cell source for regenerative medicine or the like after chromosomes are analyzed.
That is, according to the present invention, as in the related art, visual chromosome observation using a microscope is not necessary, and expertise for visual chromosome observation is not necessary. In addition, the evaluation procedure is simple and it is possible to evaluate genomic abnormalities rapidly.

[Brief Description of Drawings]

FIG. 1 is a schematic view explaining an evaluation method for genomic abnormalities in cells according to one example of the present invention.
FIG. 2 is a graph showing the change in the abundance of the indicator threonic acid over time determined by LC-MS analysis of a culture supernatant for control cells (sample C) and test cells (sample D) in an example. The horizontal axis represents the number of days of culture and the vertical axis represents area ratio. Area ratio = area value of indicator/area value of internal standard. In FIG. 3 to FIG. 8, the horizontal axis and the vertical axis are the same as above.
FIG. 3 is a graph showing the change in the abundance of the indicator cystathionine over time determined by LC-MS analysis of a culture supernatant for control cells (sample C) and test cells (sample D) in an example.
FIG. 4 is a graph showing the change in the abundance of the indicator kynurenine over time determined by LC-MS analysis of a culture supernatant for control cells (sample C) and test cells (sample D) in an example.
FIG. 5 is a graph showing the change in the abundance of the indicator cysteine over time determined by LC-MS analysis of a culture supernatant for control cells (sample C) and test cells (sample D) in an example.
FIG. 6 is a graph showing the change in the abundance of the indicator alanine over time determined by LC-MS analysis of a culture supernatant for control cells (sample C) and test cells (sample D) in an example.
FIG. 7 is a graph showing the change in the abundance of the indicator putrescine over time determined by LC-MS analysis of a culture supernatant for control cells (sample C) and test cells (sample D) in an example.
FIG. 8 is a graph showing the change in the abundance of the indicator deoxycytidine over time determined by LC-MS analysis of a culture supernatant for control cells (sample C) and test cells (sample D) in an example.
FIG. 9 is a graph showing the change in the abundance of the indicator threonic acid over time determined by LC-MS analysis of a culture supernatant for control cells (samples A, B, and C) and test cells (sample D) in an example. The horizontal axis represents the number of days of culture and the vertical axis represents area ratio. Area ratio=area value of indicator/area value of internal standard. In FIG. 10 to FIG. 15, the horizontal axis and the vertical axis are the same as above.
FIG. 10 is a graph showing the change in the abundance of the indicator kynurenine over time determined by LC-MS analysis of a culture supernatant for control cells (samples A, B, and C) and test cells (sample D) in an example.
FIG. 11 is a graph showing the change in the abundance of the indicator cysteine over time determined by LC-MS analysis of a culture supernatant for control cells (samples A, B, and C) and test cells (sample D) in an example.
FIG. 12 is a graph showing the change in the abundance of the indicator alanine over time determined by LC-MS analysis of a culture supernatant for control cells (samples A, B, and C) and test cells (sample D) in an example.
FIG. 13 is a graph showing the change in the abundance of the indicator putrescine over time determined by LC-MS analysis of a culture supernatant for control cells (samples A, B, and C) and test cells (sample D) in an example.
FIG. 14 is a graph showing the change in the abundance of the indicator deoxycytidine over time determined by LC-MS analysis of a culture supernatant for control cells (samples A, B, and C) and test cells (sample D) in an example.
FIG. 15 is a graph showing the change in the cell coverage over time determined by imaging wells for control cells (sample C) and test cells (sample D) in an example. The horizontal axis represents the number of days of culture and the vertical axis represents cell coverage.
FIG. 16 is a graph showing the change in the abundance of the indicator threonic acid over time determined by LC-MS analysis of a culture supernatant for control cells (samples A, B, and C) and test cells (sample D). The horizontal axis represents the number of days of culture and the vertical axis represents area ratio/cell coverage. Area ratio=area value of indicator/area value of internal standard. In FIG. 17 to FIG. 21, the horizontal axis and the vertical axis are the same as above.

FIG. 17 is a graph showing the change in the abundance of the indicator kynurenine over time determined by LC-MS analysis of a culture supernatant for control cells (samples A, B, and C) and test cells (sample D).

FIG. 18 is a graph showing the change in the abundance of the indicator cysteine over time determined by LC-MS analysis of a culture supernatant for control cells (samples A, B, and C) and test cells (sample D).

FIG. 19 is a graph showing the change in the abundance of the indicator alanine over time determined by LC-MS analysis of a culture supernatant for control cells (samples A, B, and C) and test cells (sample D).

FIG. 20 is a graph showing the change in the abundance of the indicator putrescine over time determined by LC-MS analysis of a culture supernatant for control cells (samples A, B, and C) and test cells (sample D).

FIG. 21 is a graph showing the change in the abundance of the indicator deoxycytidine over time determined by LC-MS analysis of a culture supernatant for control cells (samples A, B, and C) and test cells (sample D).

FIG. 22 is a graph showing $\Delta$Conc/$\Delta$Confluency in a culture supernatant of the indicator threonic acid determined by LC-MS analysis of a culture supernatant on the 3$^{rd}$ day of culture of control cells (samples A, B, and C) and test cells (sample D) in an example. The vertical axis represents $\Delta$Conc/$\Delta$Confluency. In FIG. 23 to FIG. 27, the vertical axis is the same as above.

FIG. 23 is a graph showing $\Delta$Conc/$\Delta$Confluency in a culture supernatant of the indicator kynurenine determined by LC-MS analysis of a culture supernatant on the 3$^{rd}$ day of culture of control cells (samples A, B, and C) and test cells (sample D) in an example.

FIG. 24 is a graph showing $\Delta$Conc/$\Delta$Confluency in a culture supernatant of the indicator cysteine determined by LC-MS analysis of a culture supernatant on the 3$^{rd}$ day of culture of control cells (samples A, B, and C) and test cells (sample D) in an example.

FIG. 25 is a graph showing $\Delta$Conc/$\Delta$Confluency in a culture supernatant of the indicator alanine determined by LC-MS analysis of a culture supernatant on the 3$^{rd}$ day of culture of control cells (samples A, B, and C) and test cells (sample D) in an example.

FIG. 26 is a graph showing $\Delta$Conc/$\Delta$Confluency in a culture supernatant of the indicator putrescine determined by LC-MS analysis of a culture supernatant on the 3$^{rd}$ day of culture of control cells (samples A, B, and C) and test cells (sample D) in an example.

FIG. 27 is a graph showing $\Delta$Conc/$\Delta$Confluency in a culture supernatant of the indicator deoxycytidine determined by LC-MS analysis of a culture supernatant on the 3$^{rd}$ day of culture of control cells (samples A, B, and C) and test cells (sample D) in an example.

[Description of Embodiments]

An evaluation method for genomic abnormalities of the present invention is an evaluation method for genomic abnormalities in which it is evaluated whether test cells have genomic abnormalities using pluripotent stem cells cultured in a culture medium or cells induced to differentiate from pluripotent stem cells as the test cells, including a step of measuring an abundance of an indicator in a culture supernatant of test cells; and a step of evaluating whether the test cells have genomic abnormalities based on the abundance of the indicator. The indicator, that is, a biomarker, is at least one selected from the group consisting of deoxycytidine, kynurenine, putrescine, alanine, cysteine, cystathionine, and threonic acid. In the evaluation step, it is evaluated whether the test cells have genomic abnormalities based on the abundance of the at least one indicator.

In the present invention, the test cells are pluripotent stem cells cultured in a culture medium or cells induced to differentiate from pluripotent stem cells. The pluripotent stem cells include embryonic stem cells (ES cells) induced pluripotent stem cells (iPS cells), and the like. Such pluripotent stem cells are repeatedly divided while being cultured in a culture medium and a risk of genomic abnormalities [chromosome numerical abnormalities, chromosome morphological abnormalities (abnormalities in which a part of a chromosome moves to another chromosome, and abnormalities in which the orientation of a part of a chromosome is reversed)] increases. Therefore, it is necessary to check whether cells have genomic abnormalities.

In a preferable aspect of the present invention, the evaluation method for genomic abnormalities further includes a step of measuring an abundance of an indicator in a culture supernatant of control cells known to have normal chromosomes. In this aspect, in the evaluation step, for the at least one indicator, it is evaluated whether the test cells have genomic abnormalities by comparing the abundance of the indicator in the culture supernatant of the test cells with the abundance of the corresponding indicator in the culture supernatant of the control cells.

In still another aspect of the present invention, in the evaluation method for genomic abnormalities, in place of a step of measuring an abundance of an indicator in a culture supernatant of control cells known to have normal chromosomes, abundances of indicators in a culture supernatant of control cells known to have normal chromosomes may be registered in a library in advance, and the abundance of an indicator in a culture supernatant of control cells based on this library prepared in advance may be used. In this case, the culture conditions for the test cells need to be the same as or similar to the culture conditions used for preparing the library.

Regarding a culture medium used for culturing test cells and culturing control cells, a culture medium generally used for culturing stem cells, for example, DMEM/F12 or a culture medium containing DMEM/F12 as a main component (for example, mTeSR1, TeSR-E8, and Essential-8) can be used. Table 1 shows components constituting DMEM/F12.

[Table 1]

| Components |
| --- |
| **Amino Acids** |
| Glycine |
| L-Alanine |
| L-Arginine hydrochloride |
| L-Asparagine-$H_2O$ |
| L-Aspartic acid |
| L-Cysteine hydrochloride-$H_2O$ |
| L-Cysteine 2HCl |
| L-Glutamic Acid |
| L-Glutamine |
| L-Histidine hydrochloride-$H_2O$ |
| L-Isoleucine |
| L- Leucine |
| L-Lysine hydrochloride |
| L-Methionine |
| L-Phenylalanine |
| L-Proline |
| L-Serine |
| L-Threonine |
| L-Tryptophan |
| L-Tyrosine disodium salt dihydrate |
| L-Valine |
| **Vitamins** |
| Biotin |
| Choline chloride |
| D-Calcium pantothenate |
| Folic Acid |
| Niacinamide |
| Pyridoxine hydrochloride |
| Riboflavin |
| Thiamine hydrochloride |
| Vitamin B12 |
| i-Inositol |
| **Inorganic Salts** |
| Calcium Chloride ($CaCl_2$) (anhyd.) |

(continued)

| Inorganic Salts |
| --- |
| Cupric sulfate ($CuSO_4 \cdot 5H_2O$) |
| Ferric Nitrate ($Fe(NO_3)_3 \cdot 9H_2O$) |
| Ferric sulfate ($FeSO_4 \cdot 7H_2O$) |
| Magnesium Chloride (anhydrous) |
| Magnesium Sulfate ($MgSO_4$) (anhyd.) |
| Potassium Chloride (KCl) |
| Sodium Bicarbonate ($NaHCO_3$) |
| Sodium Chloride (NaCl) |
| Sodium Phosphate dibasic ($Na_2HPO_4$) anhydrous |
| Sodium Phosphate monobasic ($NaH_2PO_4 \cdot H_2O$) |
| Zinc sulfate ($ZnSO_4 \cdot 7H_2O$ |
| **Others** |
| D-Glucose (Dextrose) |
| Hypoxanthine Na |
| Linoleic Acid |
| Lipoic Acid |
| Phenol Red |
| Putrescine 2HCl |
| Sodium Pyruvate |
| Thymidine |

In the present invention, the indicator is at least one selected from the group consisting of deoxycytidine, kynurenine, putrescine, alanine, cysteine, cystathionine, and threonic acid.

Among these indicators, amounts of deoxycytidine, kynurenine, putrescine, cystathionine or threonic acid tend to decrease when the test cells have genomic abnormalities compared with normal cells having no genomic abnormalities. On the other hand, among these indicators, amounts of alanine or cysteine tend to increase when the test cells have genomic abnormalities compared with normal cells having no genomic abnormalities.

In a preferable aspect of the present invention, in the evaluation method for genomic abnormalities, for the at least one indicator, based on determination of whether the ratio of the abundance of the indicator in the culture supernatant of the test cells to the abundance of the indicator in the culture supernatant of the control cells is at a predetermined threshold value or more, or at the predetermined threshold value or less, it is evaluated whether the test cells have genomic abnormalities.

Ratio = (abundance of the indicator in the culture supernatant of the test cells)/(abundance of the indicator in the culture supernatant of the control cells).

If the indicator is deoxycytidine, kynurenine, putrescine, cystathionine, or threonic acid, when the test cells have genomic abnormalities, the amount of the indicator tends to decrease compared with normal cells having no genomic abnormalities. Therefore, based on determination of whether the ratio is at the predetermined threshold value or less, it is possible to evaluate whether the test cells have genomic abnormalities.

If the indicator is alanine or cysteine, when the test cells have genomic abnormalities, the amount of the indicator tends to increase compared with normal cells having no genomic abnormalities. Therefore, based on determination of whether the ratio is at the predetermined threshold value or more, it is possible to evaluate whether the test cells have genomic abnormalities.

In these aspects, the threshold value can be determined by those skilled in the art in advance. The threshold value may vary depending on test cell lines to be cultured.

In the present invention, in order to allow a significant difference in the abundance between normal cells having no genomic abnormalities and cells having genomic abnormalities to be ascertained, it is recommended to select at least one selected from the group consisting of putrescine, deoxycytidine, and kynurenine among these indicators as an indicator.

In addition, in the present invention, among the indicators, it is preferable to select two or more of these indicators. In this case, when it is evaluated whether test cells have genomic abnormalities based on the abundance of two or more indicators, it is possible to perform evaluation more accurately.

In the present invention, particularly it is possible to evaluate whether there are abnormalities on the 18th chromosome.

In addition, in another aspect of the present invention, in place of cells known to have normal chromosomes, cells known to have certain atypical chromosomes may be used as control cells. In this aspect, this evaluation method for genomic abnormalities may further include a step of measuring an abundance of an indicator in a culture supernatant of control cells known to have certain atypical chromosomes. Alternatively, abundances of indicators in a culture supernatant of control cells known to have certain atypical chromosomes may be registered in a library in advance, and the abundance of an indicator in a culture supernatant of control cells based on this library prepared in advance may be used. In this case, the culture conditions for the test cells need to be the same as or similar to the culture conditions used for preparing the library.

Then, when cells known to have certain atypical chromosomes are used as control cells, in the evaluation step, for the at least one indicator, it is evaluated whether the test cells have genomic abnormalities by comparing the abundance of the indicator in the culture supernatant of the test cells with the abundance of the corresponding indicator in the culture supernatant of the control cells. That is, if the abundance of the indicator in the test cells is similar to the abundance of the corresponding indicator in the control cells, it can be determined that the test cells have the same specific abnormal chromosome as abnormal chromosomes of the control cells. If the abundance of the indicator in the test cells is different from the abundance of the corresponding indicator in the control cells, it can be determined that the test cells do not have the abnormal chromosomes of the control cells. In this case, it can be determined that the test cells have normal chromosomes or may have abnormal chromosomes different from the abnormal chromosomes of the control cells.

Regarding a method of measuring the abundance of the indicator in the culture supernatant, quantitative analysis using a gas chromatographic analysis instrument (GC), a liquid chromatographic analysis instrument (LC), or a mass spectrometer can be used, and particularly, quantitative analysis by liquid chromatography mass spectrometry (LC-MS) or gas chromatography-mass spectrometry (GC-MS) can be preferably used, but the method is not limited thereto. For example, a sample obtained by carrying out a predetermined pretreatment on a culture supernatant may be caused to flow through a column of a liquid chromatographic (LC) device together with an eluate, and the abundance of the indicator contained in the sample may be determined from results obtained by detecting components separated and eluted from the column with a detector such as an ultraviolet-visible spectroscopic detector or an infrared spectroscopic detector. In addition, a reagent that allows each of the indicators to specifically develop a color or emit light may be added to the culture supernatant, and the abundance of the indicator may be determined based on the intensity of color development or light emission.

Regarding the abundance of the indicator, in a gas chromatographic analysis method (GC), a liquid chromatographic analysis method (LC), gas chromatography-mass spectrometry (GC-MS), or liquid chromatography-mass spectrometry (LC-MS), a peak area value (area) of the indicator or a concentration of the indicator in the culture supernatant calculated from the peak area value (area) of the indicator can be used. Alternatively, "Area/Confluency," and "Concentration/Confluency" corrected (standardized) with confluency of cells; and "Area/Cell Number," and "Concentration/Cell Number" corrected with the number of cells (Cell Number, or Cell Count) may be used. When the value corrected (standardized) with the confluency of cells or the number of cells (Cell Number, or Cell Count) is used, even if there is a difference in the proliferation rates between control cells and test cells, it is possible to compare the peak area value (Area/Confluency) of the indicator or the concentration (Concentration/Confluency) of the indicator per cell number. Therefore, it is possible to perform comparison with high accuracy in which metabolisms of control cells and test cells are also taken into account, and it is possible to evaluate whether test cells have genomic abnormalities with higher accuracy.

Examples

While the present invention will be described with reference to the following examples, the present invention is not limited to the examples.

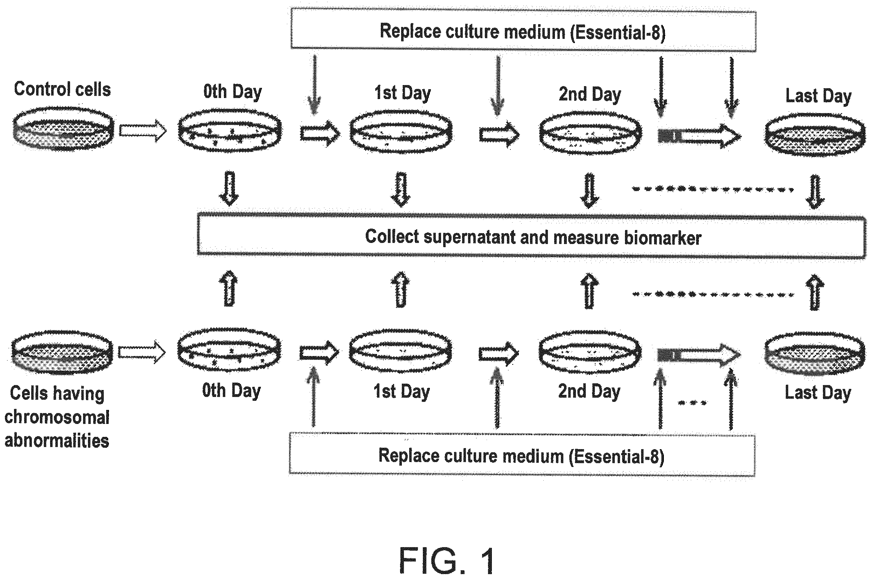

One example of evaluating whether there are genomic abnormalities in cells by the method of the present invention will be described. FIG. 1 is a schematic view explaining an evaluation method for genomic abnormalities in cells according to one example of the present invention.

In this example, four samples cultured from the same human iPS cells line [human iPS cells line samples having normal chromosomes (A, B, and C) and human iPS cells line sample having abnormalities on the 18th chromosome (D)] were used. That is, in this example, control cell samples A, B, and C from iPS cells lines having normal chromosomes and the test cell sample D from iPS cells lines having chromosomal abnormalities were used. Hereinafter, a procedure from cell culture to culture supernatant analysis in this example will be described.

[Culture of control cells and collection of culture supernatant]

The control cells were inoculated into 5 wells of a 6-well plate (well diameter of 35 mm) coated with Vitronectin-N (commercially available from Life Technologies) and cultured (in FIG. 1, only one well is shown for simplification). In addition, as a control sample, 1 well was prepared by adding only a culture medium without inoculating cells. Essential-8 was used as the culture medium, and the culture medium was replaced every day. Table 2 shows components constituting Essential-8. The day when cells were subcultured (passage) was set as the 1st day, and the culture continued until the 4th day began. After 4 days of culturing, cells were subcultured in 5 wells of a new 6-well plate (well diameter of 35 mm), and culturing was similarly continued until the 4th day began. Passage was performed a total of 3 times, and the culture supernatant collected from the culture well when the culture medium was replaced on each day was used as a sample A for analysis. A procedure from when the control cells were inoculated until subculture was completed three times was set as one set. A total of three sets of culture were performed under the same conditions and the culture supernatant collected from the culture well when the culture medium was replaced on each day was used as a sample for analysis (the culture supernatant sample collected in the second set was used as a sample B for analysis and the culture supernatant sample collected in the third set was used as a sample C for analysis).

[Table 2]

| Components |
|---|
| DMEM/F12 |
| L-ascorbic acid |
| Selenium |
| Transferrin |
| NaHCO$_3$ |
| Insulin |
| FGF2 |
| TGFβ 1 |

[Culture of test cells and collection of culture supernatant]

The test cells were inoculated into 5 wells of a 6-well plate (well diameter of 35 mm) coated with Vitronectin-N (commercially available from Life Technologies) and cultured (in FIG. 1, only one well is shown for simplification). In addition, as a control sample, 1 well was prepared by adding only a culture medium without inoculating cells. Essential-8 was used as the culture medium, and the culture medium was replaced every day. The day when cells were subcultured (passage) was set as the 1st day, and the culture continued until the 4th day began. After 4 days of culturing, cells were subcultured in 5 wells of a new 6-well plate (well diameter of 35 mm), and culturing was similarly continued until the 4th day began. Passage was performed a total of 3 times, and the culture supernatant collected from the culture well when the culture medium was replaced on each day was used as a sample D for mass spectrometry.

[Pretreatment of sample]

20 μL of a 0.5 mM isopropylmalic acid aqueous solution was added as an internal standard to 50 μL of each of the samples, and 260 μL of methanol was additionally added to modify proteins contained in the sample. Proteins were removed by a filtration filter and the filtrate was collected.

[Analysis by GC-MS]

After the samples subjected to the above pretreatment were frozen and dried and incubated in a pyridine solution containing methoxyamine hydrochloride, MSTFA (N-methyl-N-trimethylsilyltrifluoroacetamide) was additionally added to the samples, and thus compounds in the samples were trimethylsilylated. Then, these derivatized samples were subjected to analysis by GC-MS. "Smart Metabolites Database" (commercially available from Shimadzu Corporation) was used for analysis of the analysis results. This database is a collection of data obtained by analyzing various compound reference materials subjected to the same derivatization treatment as above by GC-MS. Compound identification was performed using an index indicating whether a difference between the retention index (a value relative to the retention time) set in the database and the retention index of the derivatized compound in the sample was within ±5 and whether both quantitative ions and confirmed ions present in the database were detected in the derivatized compound in the sample. On the other hand, quantification of the compound was performed by a method of calculating an area of the mass chromatogram for characteristic ions for each derivatized compound in the sample according to conditions set in the database.

[Analysis by LC-MS]

An appropriate amount of ultrapure water (Milli-Q (registered trademark) water, commercially available from Merck) was added to the samples subjected to the above pretreatment, dilution was performed, and the samples were analyzed by LC-MS. In the LC-MS analysis, compounds in the samples were temporally separated by gradient elution using a reverse phase separation column and then mass analysis was performed in a multiple reaction monitoring (MRM) mode. Analysis conditions in the MRM mode were set using a reference material for the compound. The compound was identified based on determination of whether the difference between the retention time of the reference material and the retention time of the compound in the sample was within ±0.1 minutes. In addition, the compound was quantified by a method of calculating an area of the mass chromatogram for characteristic ions for each compound in the sample.

Conditions for LC-MS/MS:

HPLC:

Device used: ultra high-performance liquid chromatograph Nexera X2 series (commercially available from Shimadzu Corporation)
Column: Discovery HS F5-3* (2.1 mm I.D.×150 mmL, particle size: 3 μm, Sigma-Aldrich) LC/MS:
Device used: ultra-high speed triple quadrupole mass spectrometer LCMS-8060 (commercially available from Shimadzu Corporation)
In addition to the analysis by GC-MS and the analysis by LC-MS, analysis can also be performed by LC, and using the results, according to the same procedures as below, data analysis can be performed and genomic abnormalities can be evaluated.

[Measurement of cell coverage]

The well was imaged on each culture day using Biostudio (commercially available from Nikon), and an area proportion of cells in the well was calculated as a cell coverage.

[Data analysis 1: area ratio of indicator]

For the culture supernatant samples A, B, C, and D collected on each culture day, a value (area ratio) was calculated by dividing the quantitative value (area value) of each indicator compound (marker) determined by performing the above LC-MS analysis by the quantitative value (area value) of the internal standard. Then, for each of the control cells and the test cells, a value obtained by subtracting the area ratio of the control sample from the area ratio obtained from the results obtained by analyzing the culture supernatant collected from the 5 wells by LC-MS was calculated, and additionally, the corrected value corrected with a cell coverage was compared as an index value of the amount of substance produced or consumed by one cell during 24 hour-culture. Specifically, when the value obtained by subtracting the area ratio of the control sample from the area ratio determined for the control cells was set as [a], the value obtained by subtracting the area ratio of the control sample from the area ratio determined for the test cells was set as [b], the rate of increase in the cell coverage of target cells for every 24-hour culture was set as [c], and the rate of increase in the cell coverage of test cells for every 24-hour culture was set as [d], if confidence intervals of [a]/[c] and [b]/[d] did not overlap, it was determined that there was no significant difference in the amount of substance produced or consumed by one cell during 24-hour culture between the culture supernatant of the control cells and the culture supernatant of the test cells.

First, the results obtained from the area ratio are shown below.

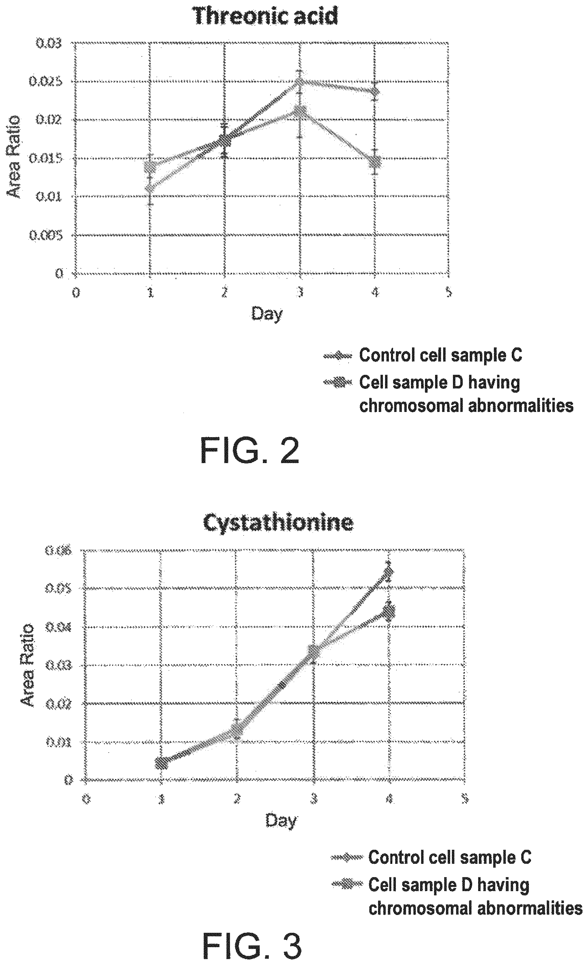

FIG. 2 is a graph showing the change in the abundance of the indicator threonic acid over time determined by LC-MS analysis of a culture supernatant for control cells (sample C) and test cells (sample D). The horizontal axis represents the number of days of culture and the vertical axis represents area ratio. Area ratio=area value of indicator/area value of internal standard. In FIG. 3 to FIG. 8, the horizontal axis and the vertical axis are the same as above.

FIG. 3 is a graph showing the change in the abundance of the indicator cystathionine over time determined by LC-MS analysis of a culture supernatant for control cells (sample C) and test cells (sample D).

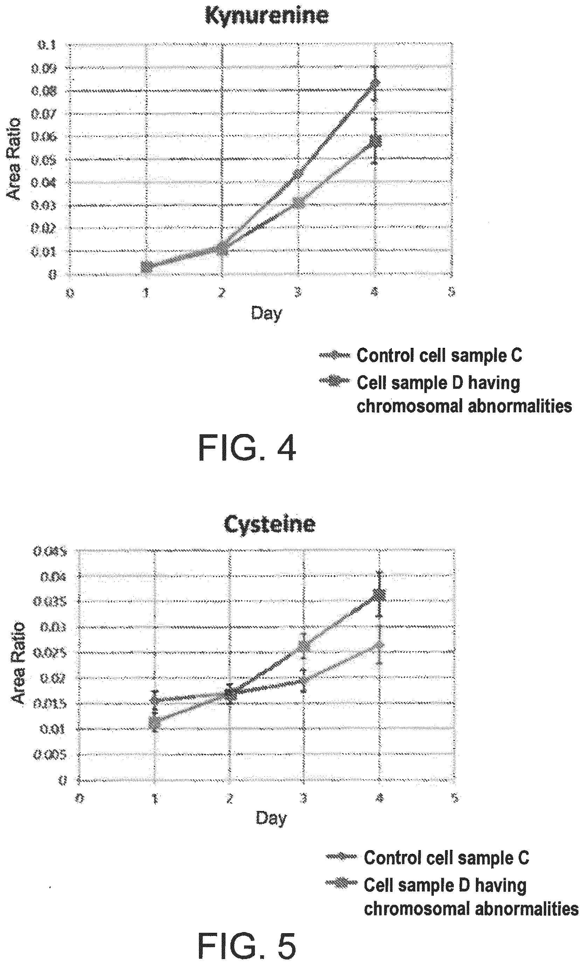

FIG. 4 is a graph showing the change in the abundance of the indicator kynurenine over time determined by LC-MS analysis of a culture supernatant for control cells (sample C) and test cells (sample D).

FIG. 5 is a graph showing the change in the abundance of the indicator cysteine over time determined by LC-MS analysis of a culture supernatant for control cells (sample C) and test cells (sample D).

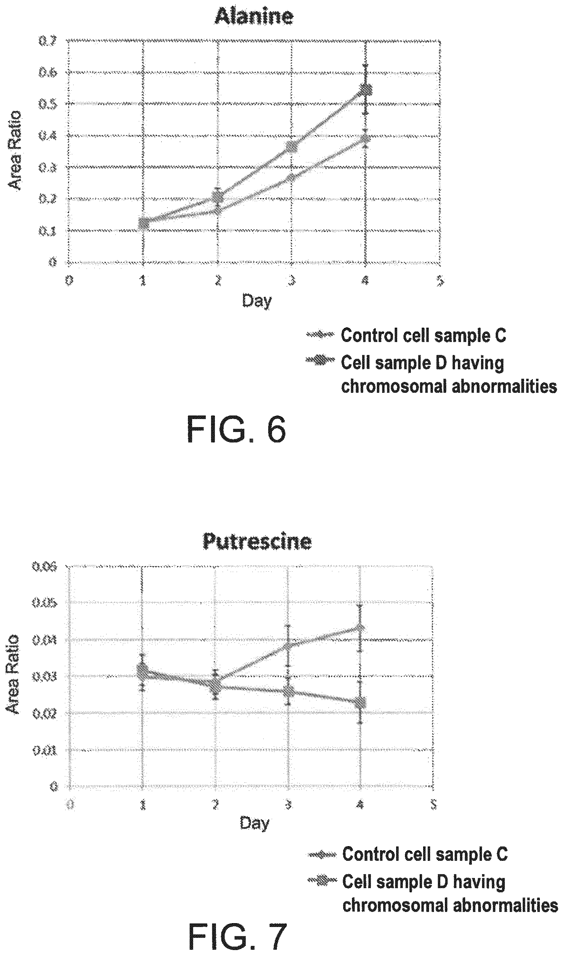

FIG. 6 is a graph showing the change in the abundance of the indicator alanine over time determined by LC-MS analysis of a culture supernatant for control cells (sample C) and test cells (sample D).

FIG. 7 is a graph showing the change in the abundance of the indicator putrescine over time determined by LC-MS analysis of a culture supernatant for control cells (sample C) and test cells (sample D).

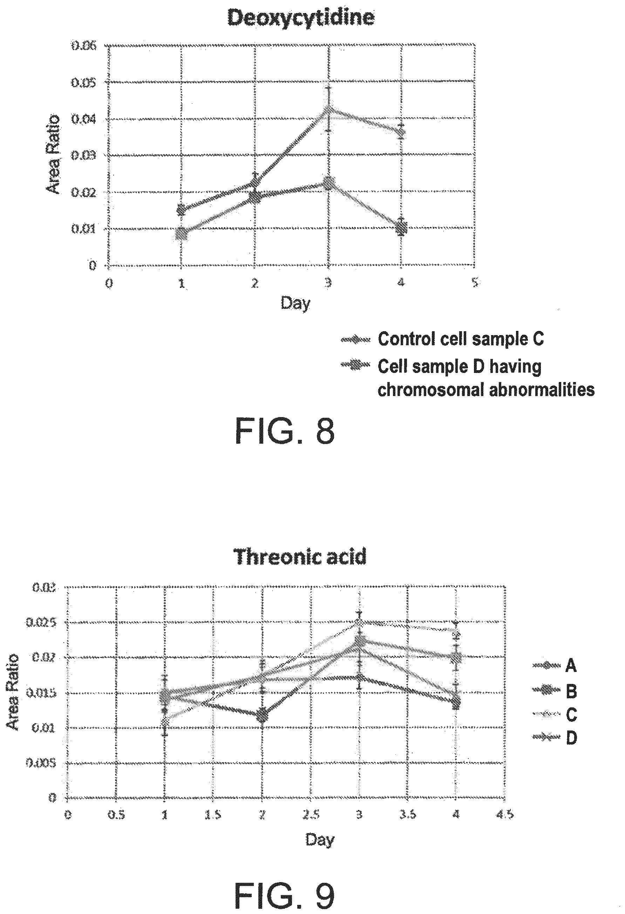

FIG. 8 is a graph showing the change in the abundance of the indicator deoxycytidine over time determined by LC-MS analysis of a culture supernatant for control cells (sample C) and test cells (sample D).

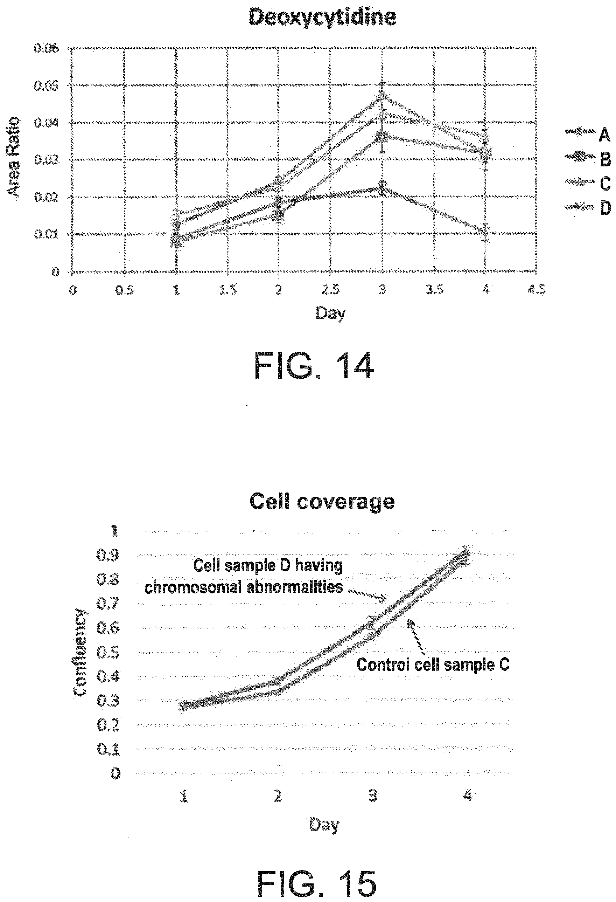

Here, the control cells (sample C) and the test cells (sample D) had substantially the same cell proliferation rate in culturing, that is, the numbers of cells over time were substantially the same. FIG. 15 is a graph showing the change in the cell coverage over time determined by imaging wells for control cells (sample C) and test cells (sample D). The horizontal axis represents the number of days of culture and the vertical axis represents cell coverage (confluency). In FIG. 15, it can be understood that cell proliferation curves of the control cells (sample C) and the test cells (sample D) in culturing were substantially the same. Regarding the sample of control cells, a sample having substantially the same cell proliferation curve as the sample of test cells was preferable. If the cell proliferation curves were substantially the same, since the numbers of cells were substantially the same, it was possible to perform comparison with the peak area value (area) of the indicator per cell number or the concentration of the indicator. Therefore, it was possible to perform comparison with high accuracy in which metabolisms of the control cells and the test cells were also taken into account, and it was possible to evaluate whether test cells had genomic abnormalities with higher accuracy.

In FIG. 2 to FIG. 4, and FIG. 7 to FIG. 8, it was understood that the amounts of the indicators threonic acid, cystathionine, kynurenine, putrescine, and deoxycytidine significantly reduced over time in the test cells having abnormalities on the 18th chromosome (sample D) compared with the control cells having normal chromosomes (sample C).

On the other hand, in FIG. 5 to FIG. 6, it was found that the amounts of the indicators cysteine and alanine significantly increased over time in the test cells having abnormalities on the 18th chromosome (sample D) compared with the control cells having normal chromosomes (sample C).

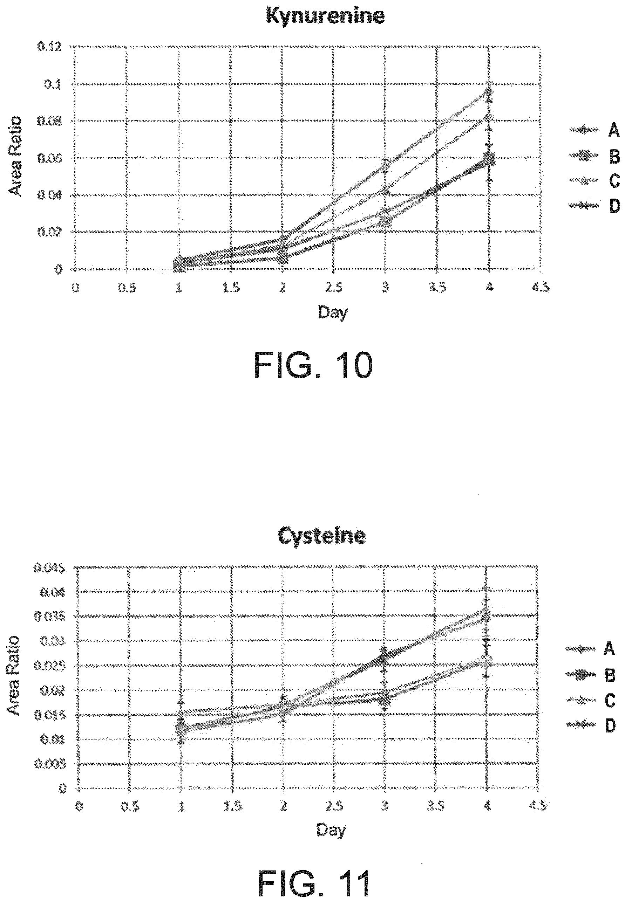

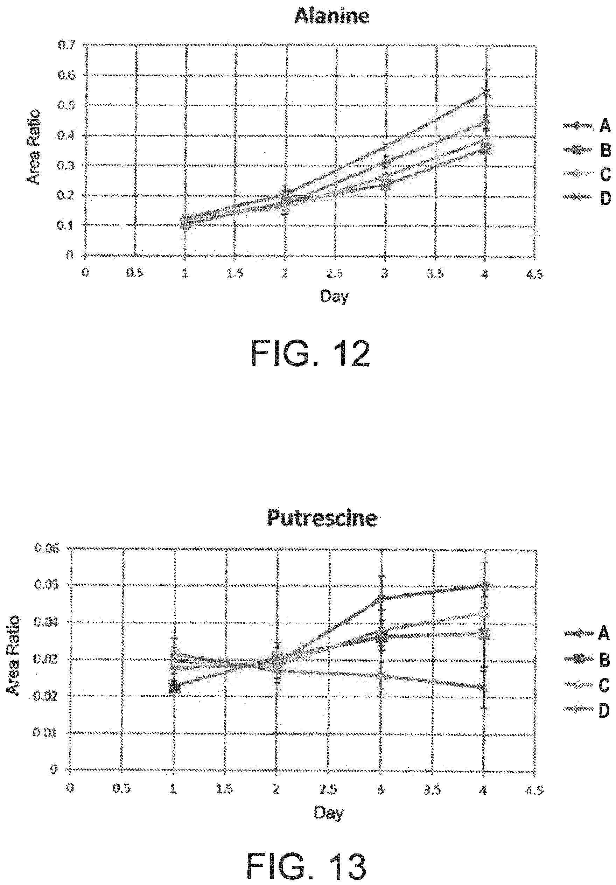

FIG. 9 to FIG. 14 show the results of the control cells (sample A) and the control cells (sample B) that were written and added in addition to the control cells (sample C) and the test cells (sample D) with respect to the above FIG. 2 and FIG. 4 to FIG. 8. The control cells (sample A) and the control cells (sample B) had different cell proliferation rates in culturing from the control cells (sample C) and the test cells (sample D), that is, the numbers of cells over time were different. However, there was a significant difference in the abundance of each indicator between the control cells (sample A to C) and the test cells (sample D). Therefore, even if the cell proliferation rates in culturing were different, it was possible to evaluate whether test cells had genomic abnormalities by comparing the abundances of each indicator in the control cells and the test cells.

Next, the results obtained from a corrected value (Area ratio/Confluency) determined by correcting the value of the area ratio with the cell coverage (confluency) are shown below.

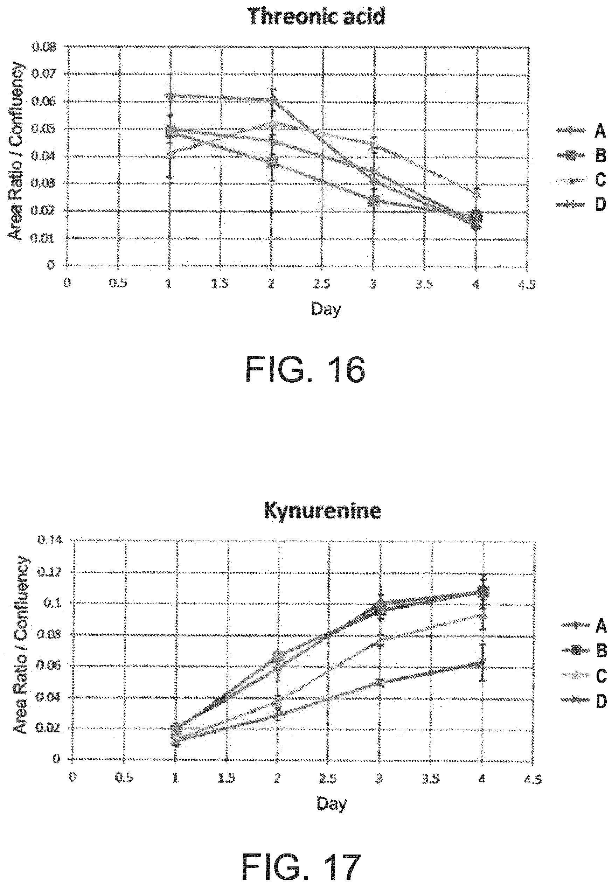

FIG. 16 is a graph showing the change in the abundance of the indicator threonic acid over time determined by LC-MS analysis of a culture supernatant for control cells (samples A, B, and C) and test cells (sample D). The horizontal axis represents the number of days of culture and the vertical axis represents area ratio/cell coverage. Area ratio=area value of indicator/area value of internal standard. In FIG. 17 to FIG. 21, the horizontal axis and the vertical axis are the same as above.

FIG. 17 is a graph showing the change in the abundance of the indicator kynurenine over time determined by LC-MS analysis of a culture supernatant for control cells (samples A, B, and C) and test cells (sample D).

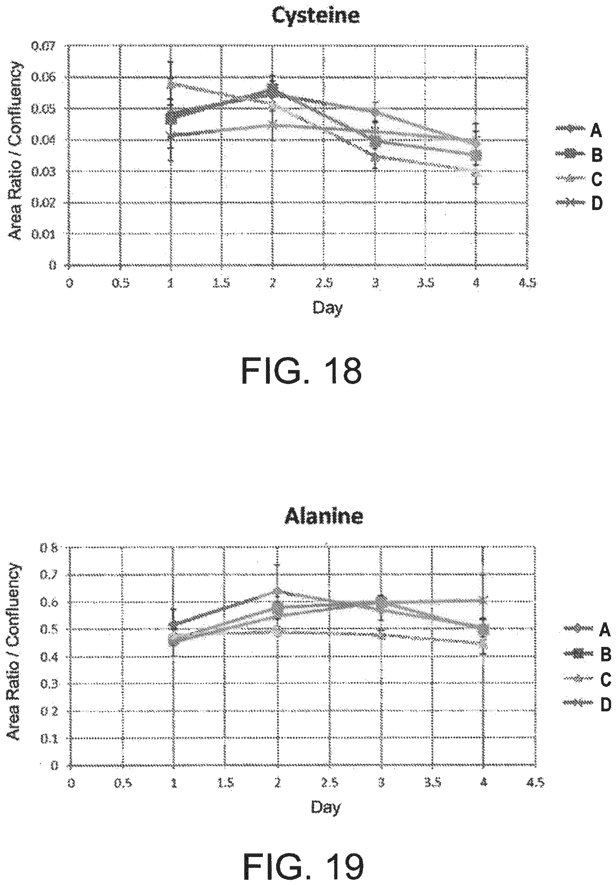

FIG. 18 is a graph showing the change in the abundance of the indicator cysteine over time determined by LC-MS analysis of a culture supernatant for control cells (samples A, B, and C) and test cells (sample D).

FIG. 19 is a graph showing the change in the abundance of the indicator alanine over time determined by LC-MS analysis of a culture supernatant for control cells (samples A, B, and C) and test cells (sample D).

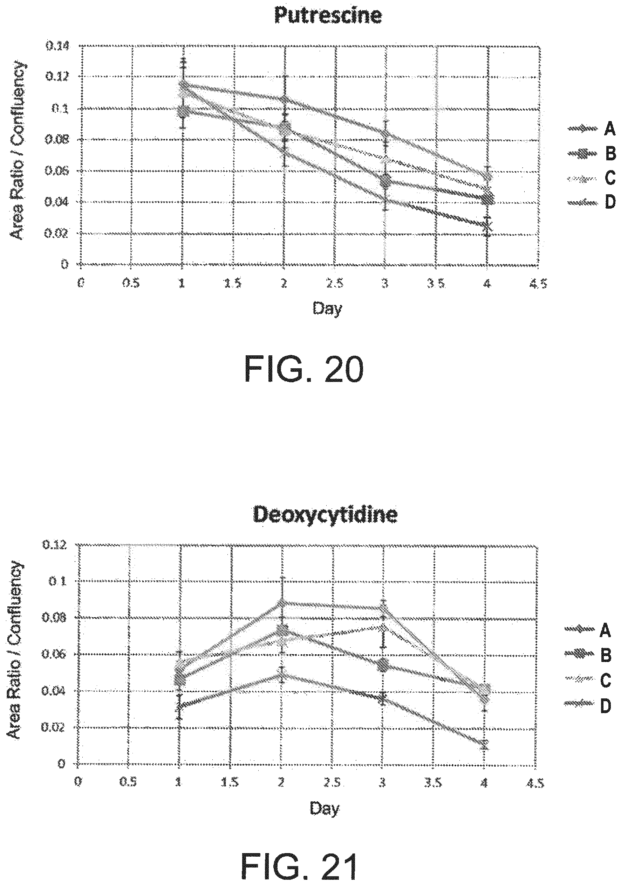

FIG. 20 is a graph showing the change in the abundance of the indicator putrescine over time determined by LC-MS analysis of a culture supernatant for control cells (samples A, B, and C) and test cells (sample D).

FIG. 21 is a graph showing the change in the abundance of the indicator deoxycytidine over time determined by LC-MS analysis of a culture supernatant for control cells (samples A, B, and C) and test cells (sample D).

Here, as described above, in FIG. 15, the control cells (sample C) and the test cells (sample D) had substantially the same cell proliferation rate in culturing, that is, the numbers of cells over time were substantially the same.

In FIG. 16, FIG. 17, FIG. 20 and FIG. 21, it was understood that the amounts of the indicators threonic acid, kynurenine, putrescine, and deoxycytidine significantly reduced over time in the test cells having abnormalities on the 18th chromosome (sample D) compared with the control cells having normal chromosomes (sample C).

On the other hand, in FIG. 18 and FIG. 19, it was understood that the amounts of the indicators cysteine and alanine significantly increased over time in the test cells having abnormalities on the 18th chromosome (sample D) compared with the control cells having normal chromosomes (sample C).

A method of calculating a confidence interval of ΔArea ratio/ΔConfluency is as follows.

$$\Delta\text{Area ratio} = (\text{area ratio determined for control cells on the } 3^{\text{rd}} \text{ day}) - (\text{area ratio of control sample on the } 3^{\text{rd}} \text{ day})$$

$$\Delta\text{Confluency} = (\text{cell coverage determined for control cell on the } 3^{\text{rd}} \text{ day}) - (\text{cell coverage determined for control cells C on the } 2^{\text{nd}} \text{ day})$$

Here, for the normal control cells, the confidence interval was calculated using all the measured values of the target cells (samples A, B, and C).

Table 3 shows the 95% confidence interval for the indicators, and Table 4 shows the 90% confidence interval for the indicators.

[Table 3]

| 95% confidence interval | | | | | | | | | |
|---|---|---|---|---|---|---|---|---|---|
| | | | Threonic acid | Cystathionine | Cysteine | Alanine | Putrescine | Deoxycytidine | Kynurenine |
| Normal type | 95% confidence interval (lower limit value) | | 0.0966 | 0.1322 | 0.0546 | 1.0769 | 0.1192 | 0.1860 | 0.1877 |
| | 95% confidence interval (upper limit value) | | 0.1287 | 0.1529 | 0.0700 | 1.2625 | 0.1435 | 0.2310 | 0.2147 |
| Abnormal type | 95% confidence interval (lower limit value) | | 0.0690 | 0.1129 | 0.0734 | 1.2582 | 0.0491 | 0.0926 | 0.1333 |
| | 95% confidence interval (upper limit value) | | 0.0933 | 0.1342 | 0.0880 | 1.4533 | 0.0729 | 0.1129 | 0.1506 |
| Determination | | | Good | No Good | Good | No Good | Good | Good | Good |

[Table 4]

| 90% confidence interval | | | Threonic acid | Cystathionine | Cysteine | Alanine | Putrescine | Deoxycytidine | Kynurenine |
|---|---|---|---|---|---|---|---|---|---|
| Normal type | 90% confidence interval (lower limit value) | | 0.0992 | 0.1339 | 0.0559 | 1.0921 | 0.1212 | 0.1897 | 0.1899 |
| | 90% confidence interval (upper limit value) | | 0.1261 | 0.1512 | 0.0687 | 1.2473 | 0.1415 | 0.2273 | 0.2125 |
| Abnormal type | 90% confidence interval (lower limit value) | | 0.0712 | 0.1148 | 0.0747 | 1.2757 | 0.0512 | 0.0944 | 0.1348 |
| | 90% confidence interval (upper limit value) | | 0.0912 | 0.1323 | 0.0867 | 1.4359 | 0.0708 | 0.111 | 0.1491 |
| Determination | | | Good | Good | Good | Good | Good | Good | Good |

In Table 3 and Table 4, when the value obtained by subtracting the area ratio of the control sample from the area ratio determined for the control cell samples A, B, and C was set as [a], the value obtained by subtracting the area ratio of the control sample from the area ratio determined for the test cells was set as [b], the rate of increase in the cell coverage of target cells for every 24-hour culture was set as [c], and the rate of increase in the cell coverage of test cells for every 24-hour culture was set as [d], if confidence intervals of [a]/[c] and [b]/[d] did not overlap, it was determined that there was a significant difference in the amount of substance produced or consumed by one cell during 24-hour culture between the culture supernatant of the control cells and the culture supernatant of the test cells. When such a determination was performed, more accurate determination was possible.

[Data analysis 2: concentration ratio of indicator]

For the culture supernatant samples collected on each culture day, regarding the area value of each indicator compound (marker) determined by performing the above LC-MS analysis, from the LC-MS analysis results of the indicator compounds with known concentrations, the concentration value of each indicator compound was calculated by an external standard method. Then, for each of the control cell samples and test cell samples, a value (ΔConc) obtained by subtracting the concentration value of the control sample from the concentration value obtained from the results obtained by analyzing the culture supernatant collected from the 5 wells by LC-MS was calculated, and additionally, the corrected value (ΔConc/ΔConfluency) corrected with the amount of change in the cell coverage (ΔConfluency) was compared as an index value of the amount of substance produced or consumed by one cell during 24-hour culture.

$$\Delta \mathrm{Conc} = (\text{concentration value determined for culture supernatant sample on the } 3^{rd} \text{ day}) -$$

$$(\text{concentration value of control sample on the } 3^{rd} \text{ day})$$

$$\Delta \mathrm{Confluency} = (\text{cell coverage determined for culture supernatant sample on the } 3^{rd} \text{ day}) - (\text{cell}$$

$$\text{coverage determined for culture supernatant sample on the } 2^{nd} \text{ day})$$

Here, for the normal control cell sample, the average value of ΔConc/ΔConfluency of the target cell samples A, B, and C was used.

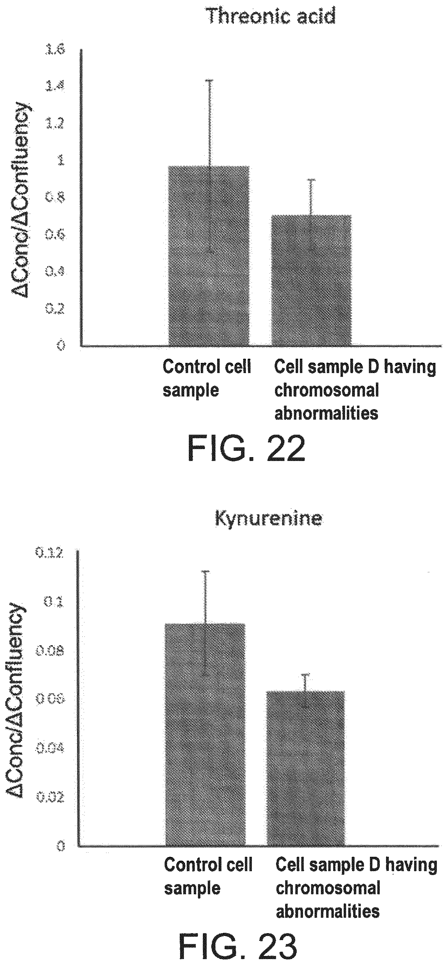

FIG. 22 is a graph showing ΔConc/ΔConfluency in a culture supernatant of the indicator threonic acid determined by LC-MS analysis of a culture supernatant on the 3rd day of culture of control cells (samples A, B, and C) and test cells (sample D). The vertical axis represents ΔConc/ΔConfluency. In FIG. 23 to FIG. 27, the vertical axis is the same as above.

FIG. 23 is a graph showing ΔConc/ΔConfluency in a culture supernatant of the indicator kynurenine determined by LC-MS analysis of a culture supernatant on the 3rd day of culture of control cells (samples A, B, and C) and test cells (sample D).

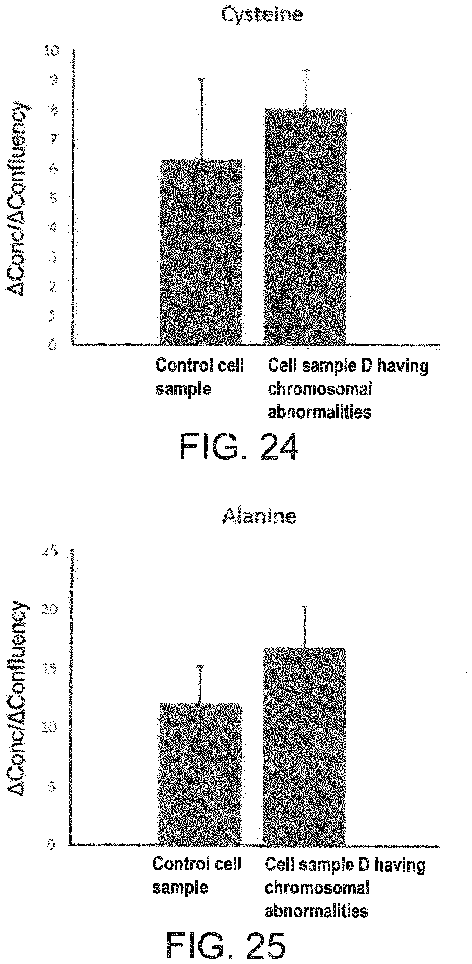

FIG. 24 is a graph showing ΔConc/ΔConfluency in a culture supernatant of the indicator cysteine determined by LC-MS analysis of a culture supernatant on the 3rd day of culture of control cells (samples A, B, and C) and test cells (sample D).

FIG. 25 is a graph showing ΔConc/ΔConfluency in a culture supernatant of the indicator alanine determined by LC-MS analysis of a culture supernatant on the 3rd day of culture of control cells (samples A, B, and C) and test cells (sample D).

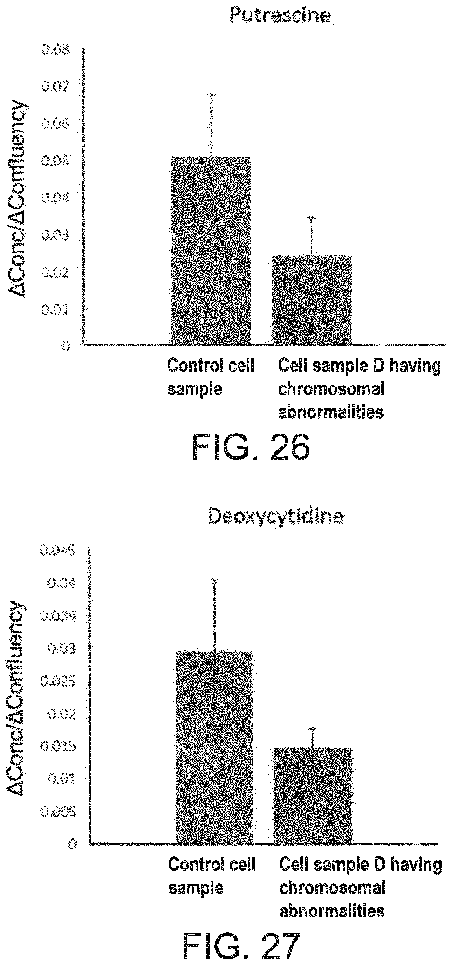

FIG. 26 is a graph showing ΔConc/ΔConfluency in a culture supernatant of the indicator putrescine determined by LC-MS analysis of a culture supernatant on the 3rd day of culture of control cells (samples A, B, and C) and test cells (sample D).

FIG. 27 is a graph showing ΔConc/ΔConfluency in a culture supernatant of the indicator deoxycytidine determined by LC-MS analysis of a culture supernatant on the 3rd day of culture of control cells (samples A, B, and C) and test cells (sample D).

Accordingly, even if comparison was performed not only with the area ratio but also the concentration ratio, similarly, the results in which there was a significant difference between normal cells and cells having abnormalities were obtained.

In the above example, a case in which cell lines A, B, and C were used as control cells and the cell lines D were used as test cells has been described. The present invention can also be applied to a case in which other cell lines are used as control cells and other cell lines are used as test cells. This will be understood by those skilled in the art that, according to the above example, the indicator (that is, the marker) can be used for determining genomic abnormalities. In addition, it can be clearly understood that the culture medium for cells is not limited to those used in the example, but various culture mediums can be used.

When a process of selecting the test cells is performed based on the results of evaluating genomic abnormalities by the method of the present invention, only normal cells can be continuously cultured in a non-invasive manner.

**Claims**

1. An evaluation method for genomic abnormalities, wherein it is evaluated whether test cells have genomic abnormalities using pluripotent stem cells cultured in a culture medium or cells induced to differentiate from pluripotent stem cells as the test cells, the method comprising:

   a step of measuring an abundance of an indicator in a culture supernatant of test cells; and
   a step of evaluating whether the test cells have genomic abnormalities based on the abundance of the indicator, wherein the indicator is at least one selected from the group consisting of deoxycytidine, kynurenine, putrescine, alanine, cysteine, cystathionine, and threonic acid, and
   wherein it is evaluated whether the test cells have genomic abnormalities based on the abundance of the at least one indicator.

2. The evaluation method for genomic abnormalities according to claim 1, further comprising
   a step of measuring an abundance of the indicator in a culture supernatant of control cells known to have normal chromosomes,
   wherein, for the at least one indicator, it is evaluated whether the test cells have genomic abnormalities by comparing the abundance of the indicator in the culture supernatant of the test cells with the abundance of the indicator in the culture supernatant of the control cells.

3. The evaluation method for genomic abnormalities according to claim 2,
   wherein, for the at least one indicator, based on determination of whether the ratio of the abundance of the indicator in the culture supernatant of the test cells to the abundance of the indicator in the culture supernatant of the control cells is at a predetermined threshold value or more, or at the predetermined threshold value or less, it is evaluated whether the test cells have genomic abnormalities.

4. The evaluation method for genomic abnormalities according to claim 3,
   wherein, when the indicator is deoxycytidine, kynurenine, putrescine, cystathionine, or threonic acid, it is evaluated whether the test cells have genomic abnormalities based on determination of whether the ratio is at the predetermined threshold value or less.

5. The evaluation method for genomic abnormalities according to claim 3,
   wherein, when the indicator is alanine or cysteine, it is evaluated whether the test cells have genomic abnormalities based on determination of whether the ratio is at the predetermined threshold value or more.

6. The evaluation method for genomic abnormalities according to claim 1,
   wherein among the indicators, at least one selected from the group consisting of putrescine, deoxycytidine, and kynurenine is selected as the indicator.

7. The evaluation method for genomic abnormalities according to claim 1,
   wherein among the indicators, two or more of the indicators are selected.

8. The evaluation method for genomic abnormalities according to claim 1,
   wherein it is evaluated whether there are abnormalities on the 18th chromosome.

9. The evaluation method for genomic abnormalities according to claim 1,
   wherein the abundance of the indicator in the culture supernatant is evaluated by liquid chromatography-mass spectrometry (LC-MS).

10. A cell culture method, comprising a process of selecting the test cells based on the results of evaluating genomic abnormalities obtained by the method according to claim 1.

FIG. 1

## Threonic acid

FIG. 2

## Cystathionine

FIG. 3

**Kynurenine**

──►── Control cell sample C

──◆── Cell sample D having
chromosomal abnormalities

# FIG. 4

**Cysteine**

──►── Control cell sample C

──◆── Cell sample D having
chromosomal abnormalities

# FIG. 5

## Alanine

Control cell sample C

Cell sample D having chromosomal abnormalities

# FIG. 6

## Putrescine

Control cell sample C

Cell sample D having chromosomal abnormalities

# FIG. 7

**Deoxycytidine**

- Control cell sample C
- Cell sample D having chromosomal abnormalities

FIG. 8

**Threonic acid**

- A
- B
- C
- D

FIG. 9

**Kynurenine**

FIG. 10

**Cysteine**

FIG. 11

## Alanine

**FIG. 12**

## Putrescine

**FIG. 13**

FIG. 14

FIG. 15

FIG. 16

FIG. 17

FIG. 18

FIG. 19

FIG. 20

FIG. 21

Threonic acid

FIG. 22

Kynurenine

FIG. 23

28

## Cysteine

FIG. 24

## Alanine

FIG. 25

Putrescine

FIG. 26

Deoxycytidine

FIG. 27

## INTERNATIONAL SEARCH REPORT

| | International application No. |
|---|---|
| | PCT/JP2019/023962 |

**A. CLASSIFICATION OF SUBJECT MATTER**
Int.Cl. C12Q1/02(2006.01)i, C12N1/00(2006.01)i

According to International Patent Classification (IPC) or to both national classification and IPC

**B. FIELDS SEARCHED**

Minimum documentation searched (classification system followed by classification symbols)
Int.Cl. C12Q1/02, C12N1/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/REGISTRY/MEDLINE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2015/166845 A1 (SHIMADZU CORPORATION) 05 November 2015, claims 1-8, examples & US 2017/0052171 A1, claims 1-8, examples & EP 3138922 A1 & KR 10-2016-0143804 A & CN 106460028 A & JP 2018-121659 A | 1-10 |
| Y | WO 2017/068801 A1 (SHIMADZU CORPORATION) 27 April 2017, claims 1-20, examples & US 2019/0119650 A1, claims 1-20, examples & EP 3366779 A1 & CN 108350480 A | 1-10 |

☒ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 August 2019 (20.08.2019) | 27 August 2019 (27.08.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/023962

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2017/042309 A1 (INSERM (INSTITUT NATIONAL DE LA SANTE ET DE LA RECHERCHE MEDICALE)) 16 March 2017, claims 1-5, page 2, line 5 to page 3, line 6, page 4, lines 7-19, page 17, line 31 to page 18, line 2, table 1 & JP 2018-526018 A, claims 1-5, paragraphs [0004]-[0005], [0012]-[0013], [0048], table 1 & US 2018/0245037 A1 & EP 3347487 A1 & CN 108138240 A | 1-10 |
| P,X | 有田真緒 他，液体クロマトグラフ質量分析計を用いた培養上清成分分析による多能性幹細胞の非侵襲的なゲノム異常評価，　日本再生医療学会総会プログラム抄録，February 2019, vol. 18th, #P-02-001, entire text, non-official translation (ARITA, Mao et al., "Noninvasive genomic abnormality assessment of pluripotent stem cells by analysis of culture supernatant components using a liquid chromatograph-mass spectrometer", Programs and abstracts of the Congress of the Japanese Society for Regenerative Medicine) | 1-10 |
| A | JP 2007-195533 A (MINAMI, Toshiki) 09 August 2007, entire text (Family: none) | 1-10 |
| A | JP 2010-508815 A (YALE UNIVERSITY) 25 March 2010, entire text & US 2010/0120624 A1, whole document & WO 2008/066655 A2 & EP 2092074 A2 | 1-10 |
| A | JP 2012-223104 A (OLYMPUS CORP.) 15 November 2012, entire text (Family: none) | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010508815 A

- WO 2008066655 A

**Non-patent literature cited in the description**

- **LOUISE C. LAURENT.** Dynamic Changes in the Copy Number of Pluripotency and Cell Proliferation Genes in Human ES and iPS Cells during Reprogramming and Time in Culture. *Cell Stem Cell,* 07 January 2011, vol. 8, 106-118
- Screening ethnically diverse human embryonic stem cells identifies a chromosome 20 minimal amplicon conferring growth advantage. *Nature Biotechnology,* 2011, vol. 29, 1132-1144

- Guidelines for Quality Assurance of Chromosomal and Genetic Testing. The Official Journal of the Japanese Association for Chromosome and Gene Analysis. Japanese Association for Chromosome and Gene Analysis, 2014, vol. 32, 60-89